# EUROPEAN PATENT SPECIFICATION

(11) **EP 3 795 983 B1**
(45) Date of publication and mention of the grant of the patent: **07.10.2026**
(21) Application number: 19803582.6
(22) Date of filing: 20.05.2019
(51) Int. Cl.: G01N 21/65, A61B 5/00, G01N 33/497, G06N 3/04, G06N 3/08

(54) **EXHALATION-BASED LUNG CANCER DIAGNOSIS SYSTEM**
EXHALATIONSBASIERTES SYSTEM ZUR DIAGNOSE VON LUNGENKREBS
SYSTÈME DE DIAGNOSTIC DU CANCER DU POUMON FONDÉ SUR L'AIR EXPIRÉ

(30) Priority: 18.05.2018 KR 20180057191
(43) Date of publication of application: 24.03.2021
(73) Proprietor: Exopert Corporation, Seoul 02582 (KR)
(72) Inventor: CHOI, Yeon Ho, Seoul 06276 (KR); SEONG, Jun Kyung, Seoul 03001 (KR); KIM, Hyun Koo, Seoul 06579 (KR); SHIM, On, Seoul 02853 (KR); SHIN, Hyunku, Seoul 02808 (KR)
(74) Representative: dompatent
(86) International application number: PCT/KR2019/006018
(87) International publication number: WO 2019/221581

(56) References cited:
- EP-A1- 3 264 070
- EP-A1- 3 264 070
- EP-B1- 1 225 976
- WO-A1-2016/184987
- WO-A1-2017/021023
- WO-A1-2017/216794
- WO-A1-2019/221581
- WO-A1-2020/180003
- QIAO XUEZHI ET AL: "Selective Surface Enhanced Raman Scattering for Quantitative Detection of Lung Cancer Biomarkers in Superparticle@MOF Structure", vol. 30, no. 5, 1 February 2018 (2018-02-01), DE, pages 1702275, XP055872950, ISSN: 0935-9648, Retrieved from the Internet <URL:https://api.wiley.com/onlinelibrary/tdm/v1/articles/10.1002%2Fadma.201702275> DOI: 10.1002/adma.201702275
- CHRIS O PHILLIPS ET AL: "Machine learning methods on exhaled volatile organic compounds for distinguishing COPD patients from healthy controls", JOURNAL OF BREATH RESEARCH, vol. 6, no. 3, 4 July 2012 (2012-07-04), US, pages 036003, XP055692114, ISSN: 1752-7155, DOI: 10.1088/1752-7155/6/3/036003
- YUNSHENG CHEN ET AL: "Breath Analysis Based on Surface-Enhanced Raman Scattering Sensors Distinguishes Early and Advanced Gastric Cancer Patients from Healthy Persons", ACS NANO, vol. 10, no. 9, 26 July 2016 (2016-07-26), US, pages 8169 - 8179, XP055654111, ISSN: 1936-0851, DOI: 10.1021/acsnano.6b01441
- CHEN ET AL.: "Breath Analysis Based on Surface-Enhanced Raman Scattering Sensors Distinguishes Early and Advanced Gastric Cancer Patients from Healthy Persons", ACS NANO, vol. 10, no. 9, 13 July 2016 (2016-07-13), pages 8169 - 8179, XP055654111

## Description

### [Technical Field]

The present disclosure relates to a technology for diagnosing lung cancer based on exhaled gas.

### [Background Art]

Lung cancer is diagnosed primarily by computed tomography (CT) and biopsy which necessarily requires an invasive procedure.

However, CT has the concern of radiation exposure and is limited because the examination is possible only in large hospitals. The fact that lung cancer cannot be diagnosed conveniently and early diagnosis of lung cancer is not easy is one of the reasons why the mortality of lung cancer is high.

To overcome this advantage, a convenient and non-invasive method for diagnosing lung cancer using exhaled gas has been researched. In the exhalation-based lung cancer diagnosis method, biomarkers in breath VOCs are diagnosed by GC-MS-based analysis. This method has the following problems.

First, the result depends on the smoking, sex, etc. of the patient. The second problem is the heterogenicity of the GC-MS analysis result. The substances showing difference between lung cancer patients and healthy people were not specific individual substances such as ethyl alcohol but were a group of substances such as aldehydes, alkanes, etc. Accordingly, lung cancer-specific biomarkers in breath VOCs cannot be screened through GC-MS.

Qiao Xuezhi et al: "Selectively Surface Enhanced Raman Scattering for Quantitative Detection of Lung Cancer Biomarkers in Superparticle@MOF structure", Advanced Materials, 30, 5, 2018, 1702275 discloses capturing gaseous aldehydes with a 10 ppb limit of detection with Surface enhanced Raman scattering. WO 2017/216794 A1 discloses a method of diagnosing a disease based on the determination of levels of volatile organic compounds from a universal biomarker set, including 2-ethylhexanol, 3-methylhexane, 5-ethyl-3-methyl-octane, acetone, ethanol, ethyl acetate, ethylbenzene, isononane, isoprene, nonanal, styrene, toluene and undecane, with a selected definitive sensor set. EP 3264070 A1 discloses a system for detecting a biomarker in a sample. Additionally, Chris O Philips et al: "Machine learning methods on exhaled volatile organic compounds for distinguishing COPD patients from healthy controls", Journal of Breath Research, 6, 3, 2012, 036003 discloses machine learning approaches to diagnose chronic obstructive pulmonary disease (COPD) using breath samples collected with a portable apparatus and assayed by gas chromatography and mass spectroscopy. CHEN et al., "Breath Analysis Based on Surface-Enhanced Raman Scattering Sensors Distinguishes Early and Advanced Gastric Cancer Patients from Healthy Persons", ACS Nano, vol. 10, no. 9, 13 July 2016, pp. 8169-8179, discloses a SERS-based breath analysis system for classifying patients based on VOC signals and thus represents prior art.

### [Disclosure]

### [Technical Problem]

The present disclosure relates to an exhalation-based lung cancer diagnosis method which allows fast and accurate diagnosis of lung cancer only with the exhaled gas of a user by analyzing the difference in VOC components of exhaled gas of a lung cancer patient and a healthy person through SERS and deep learning, which is a classification method using artificial intelligence.

The technical problem to be solved by the present disclosure is not limited to that mentioned above and other technical problems not mentioned above will be clearly understood by those of ordinary skill in the art to which the present disclosure belongs from the following description.

### [Technical Solution]

The invention is set in out in the independent claim 1. Preferred embodiments are reflected in the dependent claims.

Disclosed herein but not covered by the claimed invention is an exhalation-based lung cancer diagnosis method, which includes: a step of preparing a surface-enhanced Raman spectroscopy (SERS) substrate; a step of eluting volatile organic compounds (VOCs) included in each of a plurality of cells, supplying each of the cellular VOC eluates to the SERS substrate and then measuring each of cellular SERS signals; a step of learning the signal pattern of each cell by applying deep learning to each of the cellular SERS signals; a step of collecting a patient's exhaled gas and liquefying the same using silicone oil, supplying the liquefied patient's exhaled gas to the SERS substrate and measuring an exhalation SERS signal, and then identifying the signal pattern of the exhaled gas by analyzing the exhalation SERS signal through the deep learning result; and a step of comparing and analyzing the signal pattern of the each of the cellular SERS signals and the signal pattern of the exhalation SERS signal, thereby identifying the cellular SERS signal having the highest similarity to the exhalation SERS signal, and confirming and notifying whether a lung cancer cell is present or not on the basis thereof.

The plurality of cells consists of lung cancer cells and normal cells, wherein the lung cancer cell may be at least one of A549, H2087, H446, H460, H520, H358, H441, H2170, H157, H1299, H23, Calu-3, H522, EBC1, H1650 and N417 and the normal cell may be an alveolar type II cell.

In the step of measuring each of cellular SERS signals, the VOCs included in the cell to be learned may be eluted into silicone oil by injecting silicone oil into a culture of the cell to be learned and stirring the same for a predetermined time.

In the step of measuring the exhalation SERS signal, after collecting the patient's exhaled gas into a Tedlar bag and injecting silicone oil into the Tedlar bag, the VOCs included in the patient's exhaled gas may be eluted into the silicone oil by waiting for a predetermined time.

In addition, in the step of learning the signal pattern of each cell, the signal pattern of each cellular SERS signal may be learned based on dictionary learning and, in the step of identifying the signal pattern of the exhaled gas, the signal pattern of the exhalation SERS signal may be learned based on dictionary learning.

In the step of confirming and notifying whether a lung cancer cell is present or not, the cellular SERS signal having the highest similarity may be detected through correlation analysis of the signal pattern of each cellular SERS signal and the signal pattern of the exhalation SERS signal and it may be determined whether a lung cancer cell is present or not depending on the type of the detected cellular SERS signal.

In the step of learning the signal pattern of each cell, the correlation between the cellular SERS signal and a cell type may be learned through a convolutional neural network (CNN) consisting of one input layer, a plurality of hidden layers and one output layer, and the signal pattern of each cellular SERS signal may be acquired through the CNN, and, in the step of identifying the signal pattern of the exhaled gas, the signal pattern of the exhalation SERS signal is identified through the CNN, wherein the signal pattern of each cellular SERS signal and the exhalation SERS signal is determined based on i (i is a natural number which is 2 or greater) output data of the hidden layers.

In the step of confirming and notifying whether a lung cancer cell is present or not, the cellular SERS signal having the highest similarity may be detected based on the distance between the signal pattern of each cellular SERS signal and the signal pattern of the exhalation SERS signal, and it may be confirmed whether a lung cancer cell is present or not depending on the type of the detected cellular SERS signal.

The present disclosure provides an exhalation-based lung cancer diagnosis system, which includes: a surface-enhanced Raman spectroscopy (SERS) substrate; a Raman spectrometer measuring cellular SERS signals when a volatile organic compound (VOC) eluate of each of a plurality of cells is supplied to the SERS substrate and measuring an exhalation SERS signal when an exhaled gas VOC eluate is supplied to the SERS substrate; a deep learning unit acquiring and storing the signal pattern of the cellular SERS signals by applying deep learning to the cellular SERS signals when the cellular SERS signals are measured by the Raman spectrometer and acquiring and outputting the signal pattern of the exhalation SERS signal based on the deep learning result when the exhalation SERS signal is measured; and a lung cancer diagnosis unit detecting the cellular SERS signal having the highest similarity by comparing and analyzing the signal pattern of the exhalation SERS signal and the signal pattern of the cellular SERS signals and confirming whether a lung cancer cell is present or not depending on the type of the detected cellular SERS signal.

The cellular VOC eluate can be obtained by injecting silicone oil into a culture of cells, stirring the same for a predetermined time and then separating the silicone oil into which VOCs included in the cells are eluted.

The plurality of cells consists of lung cancer cells and normal cells, wherein the lung cancer cell may be at least one of A549, H2087, H446, H460, H520, H358, H441, H2170, H157, H1299, H23, Calu-3, H522, EBC1, H1650 and N417 and the normal cell may be an alveolar type II cell.

The exhaled gas VOC eluate can be obtained by, after collecting the patient's exhaled gas into a Tedlar bag and injecting silicone oil into the Tedlar bag, waiting for a predetermined time and then separating the silicone oil into which VOCs included in the patient's exhaled gas are eluted.

The deep learning unit may acquire and store the signal pattern of an input signal through deep learning based on dictionary learning.

In addition, the deep learning unit may acquire and store the signal pattern of each input signal through deep learning based on a convolutional neural network (CNN) consisting of one input layer, a plurality of hidden layers and one output layer, and the signal pattern may be determined based on i (i is a natural number which is 2 or greater) output data of the hidden layers.

### [Advantageous Effects]

An exhalation-based lung cancer diagnosis method of the present disclosure, whereby lung cancer is diagnosed by analyzing the difference in VOC components of the exhaled gas of a lung cancer patient and a healthy person, can be performed in a non-invasive manner and can prevent the risk of radiation exposure.

In addition, the heterogenicity among patients can be overcome easily by measuring SERS of VOCs included in pure cancer cells and applying deep learning thereto, thereby diagnosing lung cancer.

Furthermore, because only about 30 minutes of time is required to liquefy the exhaled gas and measure the SERS, diagnosis can be achieved very quickly as compared to the GC-MS-based method requiring 4 hours or longer on average.

### [Brief Description of Drawings]

FIG. 1 and FIG. 2 schematically illustrate an exhalation-based lung cancer diagnosis method according to an exemplary embodiment of the present disclosure.
FIG. 3 illustrates a step of preparing a SERS substrate according to an exemplary embodiment of the present disclosure in more detail.
FIG. 4 illustrates a step of measuring a cellular SERS signal according to an exemplary embodiment of the present disclosure in more detail.
FIG. 5 shows the Raman spectra of cellular SERS signals according to an exemplary embodiment of the present disclosure.
FIG. 6 illustrates a deep learning method according to an exemplary embodiment of the present disclosure.
FIG. 7 illustrates a step of liquefying a patient's exhaled gas according to an exemplary embodiment of the present disclosure in more detail.
FIG. 8 illustrates a step of confirming whether a lung cancer cell is present or not according to an exemplary embodiment of the present disclosure in more detail.
FIG. 9 and FIG. 10 schematically illustrate an exhalation-based lung cancer diagnosis method according to another exemplary embodiment of the present disclosure.
FIG. 11 illustrates an exhalation-based lung cancer diagnosis system according to an exemplary embodiment of the present disclosure.

### [Best Mode]

The purpose and effect of the present disclosure and technical features for achieving them will become apparent by the detailed description of exemplary embodiments together with appended claims. In the following description, if it is decided that the detailed description of known function or configuration makes the subject matter of the present disclosure unclear, the detailed description will be omitted.

The terms used in the present disclosure are those defined in consideration of the functions in the present disclosure and may be changed depending on the intent, practice, etc. of users or operators.

The present disclosure is not limited by the exemplary embodiments described below and may be embodied into various different forms. Those exemplary embodiments are provided so that the present disclosure is thorough and complete, and fully conveys the concepts of the present disclosure to those of ordinary skill in the art to which the present disclosure belongs. The scope of the present disclosure is defined only be the appended claims.

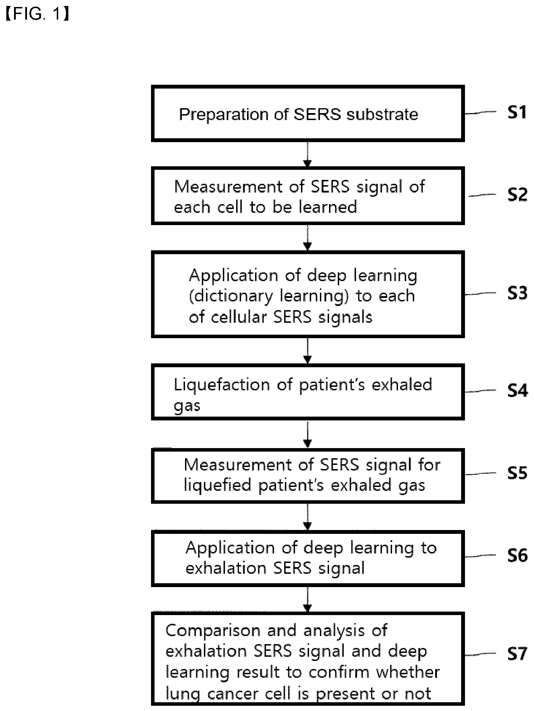

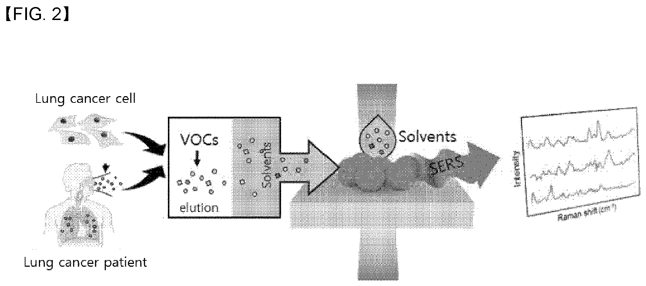

FIG. 1 and FIG. 2 schematically illustrate an exhalation-based lung cancer diagnosis method according to the present disclosure.

As shown in FIG. 1, an exhalation-based lung cancer diagnosis method of the present disclosure includes a step of preparing a surface-enhanced Raman spectroscopy (SERS) substrate (S1), a step of eluting volatile organic compounds (VOCs) included in each of the cells to be learned, spraying each eluate to the SERS substrate and then measuring a SERS signal (S2); a step of learning the specific SERS peak of each cell to be learned by applying deep learning to the SERS signal of each cell to be learned (S3), a step of collecting a patient's exhaled gas and liquefying the same using silicone oil (S4), a step of spraying the liquefied patient's exhaled gas to the SERS substrate and measuring a SERS signal (S5), and a step of confirming and notifying whether a lung cancer cell is present or not by comparing and analyzing the SERS signal of the patient's exhaled gas and the deep learning result (S6).

That is to say, the present disclosure provides a new exhalation-based method for diagnosis of lung cancer using the SERS technology and deep learning, which is a classification method using artificial intelligence.

SERS is a technique of obtaining intrinsic SERS signals occurring in response to incident light by enhancing the signals on the surface of a nanostructure. Recently, the SERS signals specific for materials are used frequently to qualitatively detect biomaterials. Formerly, the SERS technique was used mainly for liquid or solid phases and was hardly used for gas phases, because SERS is affected by molecular vibration.

Thus, in the present disclosure, VOCs included in a patient's exhaled gas are eluted into a solvent such as silicone oil and then SERS is performed using the eluate.

Also, in consideration of the difficulty of distinction from ambient factors such as smoking or ambient air quality that may occur when the patient's breath is analyzed directly, the present disclosure acquires specific VOC SERS peaks occurring in lung cancer cells and acquires and analyzes SERS signals for various lung cancer cells that can represent various actual lung cancers.

85% of lung cancer patients have non-small-cell lung cancer (NSCLC). Among them, adenocarcinoma accounts for 40%, squamous cell carcinoma accounts for 25-30%, and large-cell carcinoma accounts for 15-10%. And, 15% of lung cancer patients not belonging to NSCLC have small-cell lung cancer (SCLC).

Therefore, the present disclosure is applicable to various lung cancer cells such as A549, H2087, H446, H460, H520, H358, H441, H2170, H157, H1299, H23, Calu-3, H522, EBC1, H1650, N417, etc. and normal cells such as an alveolar type II cell, etc. In addition, various other cells that can distinguish a lung cancer patient from a healthy person may also be used.

But, for the convenience of explanation, A549, H2087, H520, H460 and H446 may be used as lung cancer cells and an alveolar type II cell may be used as a normal cell in the following description.

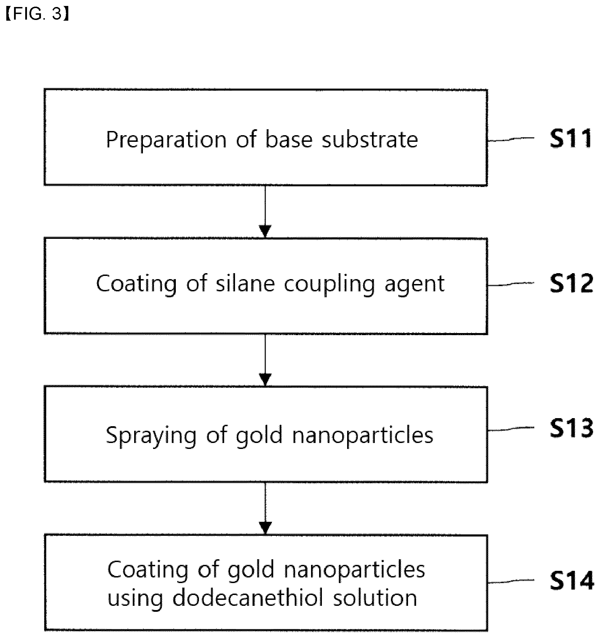

Hereinafter, the method of the present disclosure is described in more detail referring to FIGS. 3-8.

The step S1 of preparing a SERS substrate may be performed as shown in FIG. 3.

First, a base substrate is prepared (S11). The base substrate is washed after coating the surface of the base substrate with a silane coupling agent (S12). The base substrate may be embodied as a cover glass, etc. The silane coupling agent is a material for enhancing the binding between the cover glass and nanoparticles, and embodied as 0.1% 3-aminopropyltriethoxysilane (3-APTES), etc.

Then, gold nanoparticles are sprayed onto the silane coupling agent-coated surface and dried for a predetermined time (S13). After the gold nanoparticles are dried completely, the base substrate is immersed in a dodecanethiol solution for a predetermined time (e.g., for 72 hours or longer), so that the gold nanoparticles are coated on the surface (S14). Specifically, the gold nanoparticles may have a size of about 80 nm, and the dodecanethiol solution may be a 0.1% solution in an ethanol solvent. Through this process, the surface of the hydrophilic gold nanoparticles can have affinity for organic materials such as VOCs.

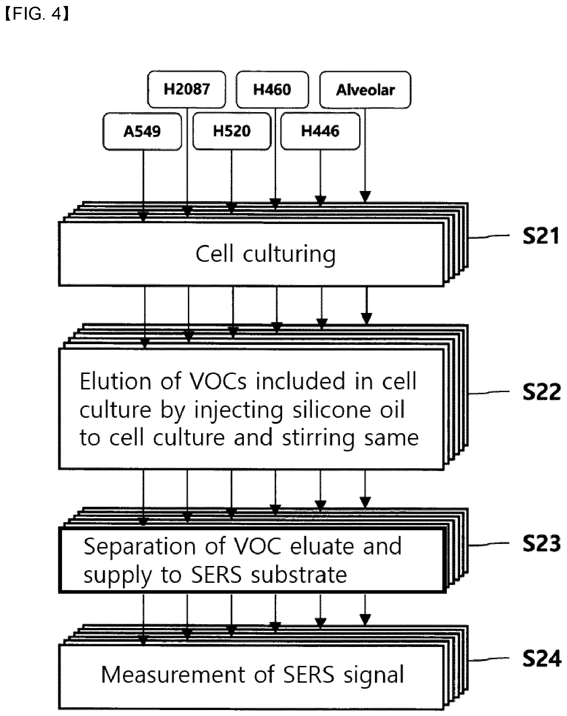

The step S2 of measuring the cellular SERS signal may be performed as shown in FIG. 4.

First, each of the cells to be learned is cultured for a predetermined time (e.g., for 72 hours) (S21). The cells to be learned consists of lung cancer cells and normal cells. The lung cancer cell may be A549, H2087, H446, H460, H520, H358, H441, H2170, H157, H1299, H23, Calu-3, H522, EBC1, H1650, N417, etc., and the normal cell may be an alveolar type II cell, etc. In addition, various other cells that can distinguish a lung cancer patient from a healthy person may also be used.

Then, the VOCs included in the cell culture are eluted by injecting silicone oil to the cell culture and stirring the layer-separated cell culture and silicone oil for a predetermined time (e.g., for 30 minutes) (S22).

Then, after separating the cell eluate and supplying to the SERS substrate (S23), the SERS signal is measured by irradiating a 785-nm laser to the SERS substrate (S24).

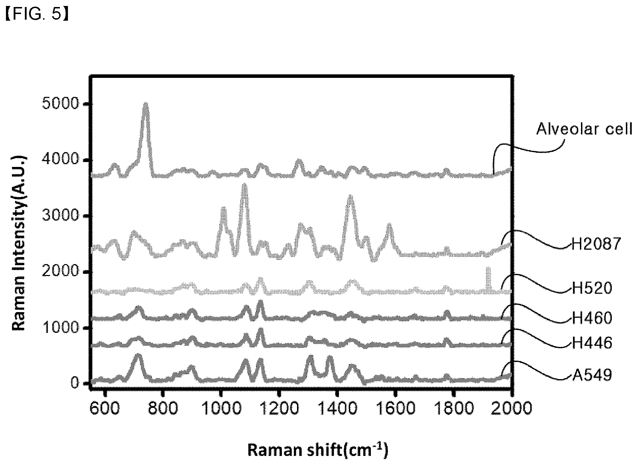

The SERS signals acquired through the step S24 may have Raman spectra shown in FIG. 5. It can be seen that the SERS signals of different cells have different signal patterns.

By measuring the VOCs of lung cancer cells and acquiring dictionary elements based thereon, i.e., by using VOCs acquired from pure cancer cells, the present disclosure aims at overcoming heterogenicity among patients.

In the step S3 of applying deep learning to the cellular SERS signal, deep learning is applied to the signal pattern (i.e., the specific SERS peak) of each cell to be learned.

Dictionary learning is an example of deep learning using artificial intelligence and is performed as follows.

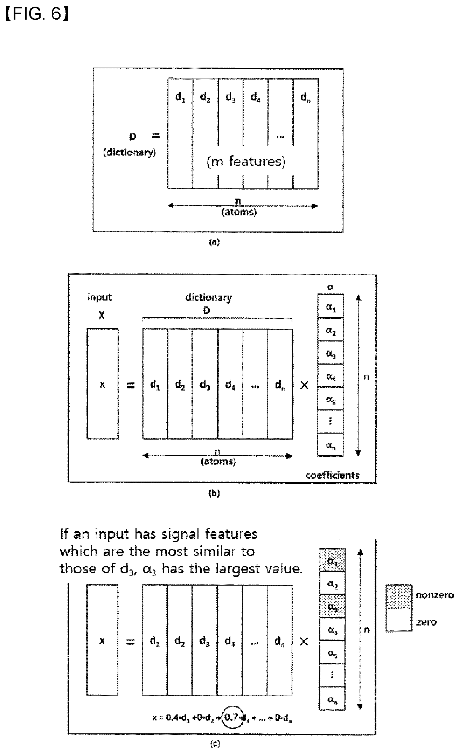

First, as shown in FIG. 6 (a), a specific database is represented by a dictionary which is a linear combination of basic elements called atoms. When a new signal (input) not existing in the database is inputted, the new signal (input) is represented by a linear combination of the atoms in the dictionary (x = α₁·d₁ + α₂·d₂ + ... + αₙ·dₙ, where w is a linear coefficient), as shown in FIG. 6 (b). Then, sparse coefficients that make most coefficients 0 are found as shown in FIG. 6 (c), and the signal having the most similar features to the new signal (input) is determined from the dictionary (D) based thereon. The atom is a row vector (m x 1) of m signal features derived from each signal included in the database, and will be referred to as a dictionary element hereinafter for the convenience of description. Dictionary learning is an example of deep learning, and the dictionary element may be generalized as the signal pattern of the SERS signal.

In the present disclosure, after applying deep learning to the SERS signals (X) of lung cancer cells and normal cells, the signal patterns of the corresponding cellular SERS signals are acquired and stored.

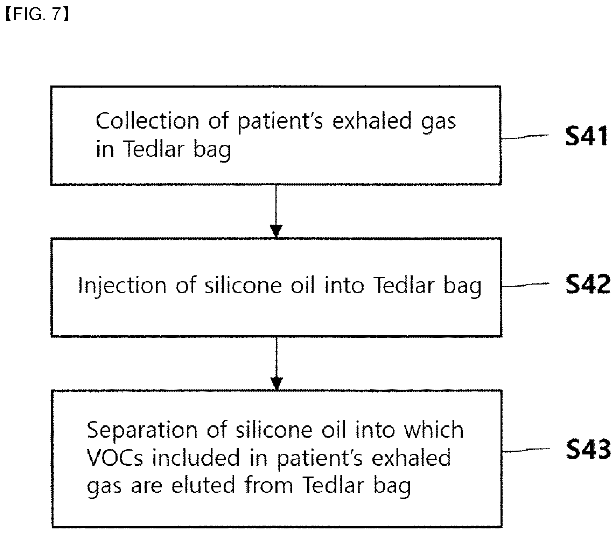

The step S4 of liquefying the patient's exhaled gas may be performed as shown in FIG. 7.

That is to say, the patient's exhaled gas is collected into a Tedlar bag (S41) and silicone oil is injected into the Tedlar bag (S42). After a predetermined time (e.g., 30 minutes) has passed, a syringe is inserted into the Tedlar bag and the silicone oil into which the VOCs included in the patient's exhaled gas have been eluted (i.e., the exhaled gas eluate) is extracted from the Tedlar bag (S43).

In the step S5 of measuring the exhalation SERS signal, the exhaled gas eluate extracted in the step S43 is supplied to the SERS substrate and then the SERS signal of the exhaled gas is measured by irradiating a 785-nm laser to the SERS substrate.

In the step S6 of applying deep learning to the exhalation SERS signal, the signal pattern of the exhaled gas (i.e., patient-derived SERS signal pattern) is acquired by applying deep learning to the exhalation SERS signal.

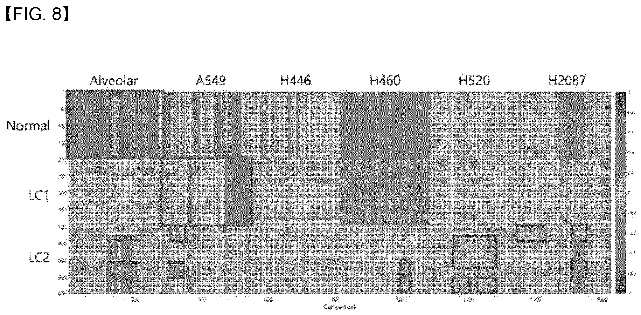

Finally, in the step S7 of confirming whether a lung cancer cell is present, the cell having the most similar signal feature to the exhalation SERS signal is detected through correlation analysis of the exhalation SERS signal and the signal pattern learned in the step S3, as shown in FIG. 8. In FIG. 8, the portion marked by a red box shows cell pairs exhibiting the highest correlation between the patient-derived SERS signal pattern and the SERS signal pattern learned through deep learning.

If the detected cell is a lung cancer cell, it is confirmed and notified that lung cancer cells exist in the body of the user. And, if the detected cell is a normal cell, it is confirmed and notified that lung cancer cells do not exist in the body of the user. Alternatively, lung cancer may be diagnosed and notified only when the detected cell is a lung cancer cell.

Although dictionary learning is described above as an example of deep learning, analysis may also be performed based on a convolutional neural network (CNN).

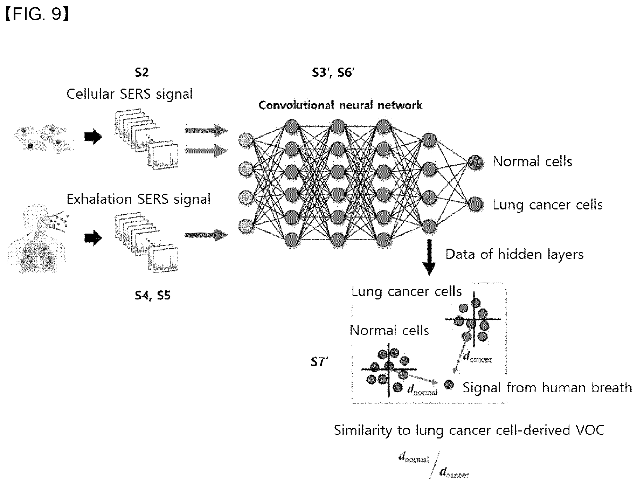

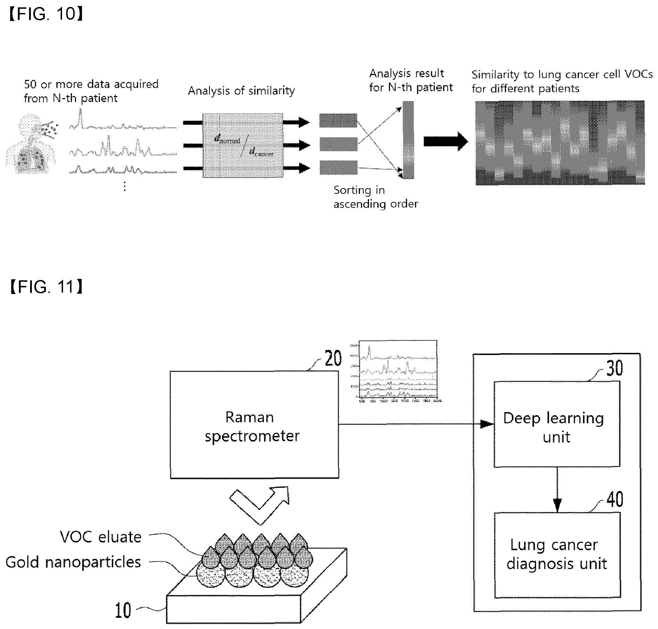

FIG. 9 and FIG. 10 schematically illustrate an exhalation-based lung cancer diagnosis method according to the present disclosure. This method is based on a CNN instead of dictionary learning. The steps related with a SERS substrate, SERS signal measurement, etc. are the same as the method based on dictionary learning and detailed description thereof will be omitted.

The CNN consists of one input layer, a plurality of hidden layers and one output layer. Neurons in each layer are connected with weights. The weights of the neurons included in each layer are adjusted by generating and learning a large number of learning data with the cellular SERS signal as input and the cell type as output.

In the present disclosure, the signal pattern of the cellular SERS signal or the exhalation SERS signal is identified using the CNN. In particular, instead of the output layer notifying the cell type, i (i is a natural number which is 2 or greater, e.g., 10) output data of the hidden layers are acquired and utilized as the signal patterns corresponding to the input signal.

First, SERS signals of lung cancer cells and normal cells (i.e., cellular SERS signals) are acquired as shown in FIG. 9.

Then, a number of learning data with the cellular SERS signal as input and the cell type as output are generated and learned iteratively by a CNN model, so that the CNN model can binarily classify input signals into lung cancer cells and normal cells. After the learning by the CNN model is completed, i (e.g., 10) hidden layer output data are acquired for each cellular SERS signal and they are stored as the signal pattern of each cellular SERS signal (S3').

After the step S3' is completed, if the exhalation SERS signal is acquired (S4, S5), the exhalation SERS signal is inputted to the learned CNN model and then i (e.g., 10) hidden layer output data are acquired and stored as the signal pattern of the exhalation SERS signal, as for the cellular SERS signal (S6').

Then, the distance between the signal pattern of each cellular SERS signal and the signal pattern of the exhalation SERS signal is computed, and the cell having the highest similarity to the exhaled gas signal is determined and notified based on the cellular SERS signal having the shortest distance value. The distance may be any one of Mahalanobis distance, Euclidean distance, cosine distance, etc., and similarity is increased in inverse proportion to the distance.

The similarity between individual exhaled gas signals can be computed through this method. Most specifically, 50-250 exhaled gas signals acquired from one person are used for analysis as shown in FIG. 10. Then, by sorting the similarity between individual exhaled gas signals in an ascending order, the similarity to lung cancer cells for different patients can be determined easily.

FIG. 11 illustrates an exhalation-based lung cancer diagnosis system according to an exemplary embodiment of the present disclosure.

Referring to FIG. 11, a system of the present disclosure may include a SERS substrate 10, a Raman spectrometer 20 measuring cellular SERS signals when a volatile organic compound (VOC) eluate of each of a plurality of cells is supplied to the SERS substrate and measuring an exhalation SERS signal when an exhaled gas VOC eluate is supplied to the SERS substrate, a deep learning unit 30 acquiring and storing the signal pattern of the cellular SERS signals by applying deep learning to the cellular SERS signals when the cellular SERS signals are measured by the Raman spectrometer and acquiring and outputting the signal pattern of the exhalation SERS signal based on the deep learning result when the exhalation SERS signal is measured, a lung cancer diagnosis unit 40 detecting the cellular SERS signal having the highest similarity by comparing and analyzing the signal pattern of the exhalation SERS signal and the signal pattern of the cellular SERS signals and confirming whether a lung cancer cell is present or not depending on the type of the detected cellular SERS signal, etc.

The cellular VOC eluate may be obtained by injecting silicone oil into a cell culture and stirring for a predetermined time, and then separating the silicone oil into which VOCs included in the cells are eluted. The exhaled gas VOC eluate can also be obtained by, after collecting the patient's exhaled gas into a Tedlar bag and injecting silicone oil into the Tedlar bag, waiting for a predetermined time and then separating the silicone oil into which VOCs included in the patient's exhaled gas are eluted.

In addition, as described above, the cells consists of lung cancer cells and normal cells, the lung cancer cell may be at least one of A549, H2087, H446, H460, H520, H358, H441, H2170, H157, H1299, H23, Calu-3, H522, EBC1, H1650 and N417, and the normal cell may be an alveolar type II cell.

That is to say, the system of the present disclosure also utilizes the above-described SERS technology and deep learning (dictionary learning, CNN), which is a classification method using artificial intelligence, and allows non-invasive diagnosis of lung cancer without the risk of radiation exposure.

In addition, although only the diagnosis of lung cancer using lung cancer cells and normal cells was described above, it will be apparent that the present disclosure can also be used for diagnosis of various cancers other than lung cancer, such as bronchial cancer, colorectal cancer, prostate cancer, breast cancer, pancreatic cancer, stomach cancer, ovarian cancer, bladder cancer, brain cancer, thyroid cancer, esophageal cancer, uterine cancer, liver cancer, kidney cancer, bile tract cancer, testicular cancer, etc., if necessary.

The foregoing description merely illustrates the exemplary embodiments of the technical idea of the present disclosure, and various modifications and changes can be made within the scope not departing from the essential feature of the present disclosure by those of ordinary skill in the art to which the present disclosure belongs. Accordingly, the exemplary embodiments disclosed in the present disclosure are for illustration, not for limiting the technical idea of the present disclosure, and the scope of the technical idea of the present disclosure is not limited by the exemplary embodiments. The invention is as defined in the appended claims.

## Claims

1. An exhalation-based lung cancer diagnosis system, comprising:
a surface-enhanced Raman spectroscopy (SERS) substrate (10);a Raman spectrometer (20) measuring cellular SERS signals when a volatile organic compound (VOC) eluate of each of a plurality of cells is supplied to the SERS substrate (10) and measuring an exhalation SERS signal when an exhaled gas VOC eluate is supplied to the SERS substrate (10);
**characterized in that**, the system further comprising:
a deep learning unit (30) trained using the cellular SERS signals and configured to acquire and store the signal pattern of the cellular SERS signals when the cellular SERS signals are measured by the Raman spectrometer (20) and to acquire and output a signal pattern of the exhalation SERS signal based on the deep learning result when the exhalation SERS signal is measured; and
a lung cancer diagnosis unit (40) configured to detect the cellular SERS signal having the highest similarity by comparing and analyzing the signal pattern of the exhalation SERS signal and the signal pattern of the cellular SERS signals and confirming whether a lung cancer cell is present or not depending on the type of the detected cellular SERS signal,
wherein the plurality of cells consists of lung cancer cells and normal cells.

2. The exhalation-based lung cancer diagnosis system according to claim 1, wherein the cellular VOC eluate can be obtained by injecting silicone oil into a culture of cells, stirring the same for a predetermined time and then separating the silicone oil into which VOCs included in the cells are eluted.

3. The exhalation-based lung cancer diagnosis system according to claim 2, wherein the lung cancer cell is at least one of A549, H2087, H446, H460, H520, H358, H441, H2170, H157, H1299, H23, Calu-3, H522, EBC1, H1650 and N417 and the normal cell is an alveolar type II cell.

4. The exhalation-based lung cancer diagnosis system according to claim 1, wherein the exhaled gas VOC eluate can be obtained by, after collecting the patient's exhaled gas into a Tedlar bag and injecting silicone oil into the Tedlar bag, waiting for a predetermined time and then separating the silicone oil into which VOCs included in the patient's exhaled gas are eluted.

5. The exhalation-based lung cancer diagnosis system according to claim 1, wherein the deep learning unit (30) acquires and stores the signal pattern of an input signal through deep learning based on dictionary learning.

6. The exhalation-based lung cancer diagnosis system according to claim 1, wherein the deep learning unit (30) acquires and stores the signal pattern of each input signal through deep learning based on a convolutional neural network (CNN) consisting of one input layer, a plurality of hidden layers and one output layer, and the signal pattern is determined based on i (i is a natural number which is 2 or greater) output data of the hidden layers.

## Patentansprüche

1. Ausatmungsbasiertes Lungenkrebs-Diagnosesystem, umfassend:
ein Substrat für oberflächenverstärkte Raman-Spektroskopie (SERS-Substrat) (10);ein Raman-Spektrometer (20), das zelluläre SERS-Signale misst, wenn dem SERS-Substrat (10) ein Eluat einer flüchtigen organischen Verbindung (VOC-Eluat) von jeder einer Vielzahl von Zellen zugeführt wird, und das ein Ausatmungs-SERS-Signal misst, wenn dem SERS-Substrat (10) ein VOC-Eluat eines ausgeatmeten Gases zugeführt wird;
**dadurch gekennzeichnet, dass** das System ferner Folgendes umfasst:
eine Deep-Learning-Einheit (30), die anhand der zellulären SERS-Signale trainiert wird und so konfiguriert ist, dass sie das Signalmuster der zellulären SERS-Signale erfasst und speichert, wenn durch das Raman-Spektrometer (20) die zellulären SERS-Signale gemessen werden, und dass sie auf der Basis des Deep-Learning-Ergebnisses ein Signalmuster des Ausatmungs-SERS-Signals erfasst und ausgibt, wenn das Ausatmungs-SERS-Signal gemessen wird; und
eine Lungenkrebs-Diagnoseeinheit (40), die so konfiguriert ist, dass sie das zelluläre SERS-Signal mit der größten Ähnlichkeit nachweisen kann, indem sie das Signalmuster des Ausatmungs-SERS-Signals und das Signalmuster der zellulären SERS-Signale miteinander vergleicht und analysiert und je nach Art des nachgewiesenen SERS-Signals bestätigt, ob eine Lungenkrebszelle vorhanden ist oder nicht,
wobei die Vielzahl der Zellen aus Lungenkrebszellen und normalen Zellen besteht.

2. Ausatmungsbasiertes Lungenkrebs-Diagnosesystem gemäß Anspruch 1, wobei das zelluläre VOC-Eluat dadurch erhalten werden kann, dass man Silikonöl in eine Kultur von Zellen injiziert, dieselbe während einer vorbestimmten Zeitdauer rührt und dann das Silikonöl, in das in den Zellen vorhandene VOCs eluiert sind, abtrennt.

3. Ausatmungsbasiertes Lungenkrebs-Diagnosesystem gemäß Anspruch 2, wobei die Lungenkrebszelle wenigstens eine aus A549, H2087, H446, H460, H520, H358, H441, H2170, H157, H1299, H23, Calu-3, H522, EBC1, H1650 und N417 ist und die normale Zelle ein Pneumozyt Typ II ist.

4. Ausatmungsbasiertes Lungenkrebs-Diagnosesystem gemäß Anspruch 1, wobei das VOC-Eluat des ausgeatmeten Gases dadurch erhalten werden kann, dass man nach dem Auffangen des von dem Patienten in einen Tedlar-Beutel ausgeatmeten Gases und Injizieren von Silikonöl in den Tedlar-Beutel während einer vorbestimmten Zeitdauer wartet und dann das Silikonöl, in das im von dem Patienten ausgeatmeten Gas vorhandene VOCs eluiert sind, abtrennt.

5. Ausatmungsbasiertes Lungenkrebs-Diagnosesystem gemäß Anspruch 1, wobei die Deep-Learning-Einheit (30) das Signalmuster eines Eingangssignals durch Deep Learning auf der Basis von Dictionary-Learning erfasst und speichert.

6. Ausatmungsbasiertes Lungenkrebs-Diagnosesystem gemäß Anspruch 1, wobei die Deep-Learning-Einheit (30) das Signalmuster jedes Eingangssignals durch Deep Learning auf der Basis eines Convolutional Neural Network (CNN), das aus genau einer Eingabeschicht, einer Vielzahl von verborgenen Schichten und genau einer Ausgabeschicht besteht, erfasst und speichert, und das Signalmuster auf der Basis von i (wobei i eine natürliche Zahl größer oder gleich 2 ist) Ausgabedaten der verborgenen Schichten bestimmt wird.

## Revendications

1. Système de diagnostic du cancer du poumon basé sur l'expiration, comprenant :
un substrat (10) pour la spectroscopie Raman exaltée par effet de surface (SERS) ;un spectromètre Raman (20) qui mesure des signaux SERS cellulaires, lorsqu'un éluat de composé organique volatil (COV) de chacune d'une pluralité de cellules est fourni au substrat SERS (10), et qui mesure un signal SERS d'expiration, lorsqu'un éluat de COV d'un gaz expiré est fourni au substrat SERS (10) ;
**caractérisé en ce que** ledit système comprend en outre :
une unité d'apprentissage profond (30) entraînée à l'aide des signaux SERS cellulaires et configurée pour acquérir et mémoriser le motif de signal des signaux SERS cellulaires, lorsque les signaux SERS cellulaires sont mesurés par le spectromètre Raman (20), et pour acquérir et produire un motif de signal du signal SERS d'expiration sur la base du résultat de l'apprentissage profond, lorsque le signal SERS d'expiration est mesuré ; et
une unité de diagnostic du cancer du poumon (40) configurée pour détecter le signal SERS cellulaire présentant la plus grande similarité en comparant et analysant le motif de signal du signal SERS d'expiration et le motif de signal des signaux SERS cellulaires, et confirmant la présence ou l'absence d'une cellule cancéreuse du poumon en fonction du type de signal SERS cellulaire détecté,
ladite pluralité de cellules consistant en des cellules cancéreuses du poumon et des cellules normales.

2. Système de diagnostic du cancer du poumon basé sur l'expiration selon la revendication 1, dans lequel l'éluat de COV cellulaire peut être obtenu en injectant de l'huile de silicone dans une culture de cellules, en agitant le tout pendant une durée prédéterminée, puis en séparant l'huile de silicone dans laquelle sont élués les COV contenus dans les cellules.

3. Système de diagnostic du cancer du poumon basé sur l'expiration selon la revendication 2, dans lequel ladite cellule cancéreuse du poumon est au moins l'une parmi A549, H2087, H446, H460, H520, H358, H441, H2170, H157, H1299, H23, Calu-3, H522, EBC1, H1650 et N417, et ladite cellule normale est un pneumocyte de type II.

4. Système de diagnostic du cancer du poumon basé sur l'expiration selon la revendication 1, dans lequel l'éluat de COV du gaz expiré peut être obtenu en recueillant le gaz expiré du patient dans un sac Tedlar et en injectant de l'huile de silicone dans le sac Tedlar, puis en attendant un temps prédéterminé, ensuite en séparant l'huile de silicone dans laquelle sont élués les COV contenus dans le gaz expiré du patient.

5. Système de diagnostic du cancer du poumon basé sur l'expiration selon la revendication 1, dans lequel l'unité d'apprentissage profond (30) acquiert et mémorise le motif de signal d'un signal d'entrée par apprentissage profond basé sur l'apprentissage de dictionnaire.

6. Système de diagnostic du cancer du poumon basé sur l'expiration selon la revendication 1, dans lequel l'unité d'apprentissage profond (30) acquiert et mémorise le motif de signal de chaque signal d'entrée par apprentissage profond basé sur un réseau de neurones à convolution (CNN) composé d'une couche d'entrée, d'une pluralité de couches cachées et d'une couche de sortie, et le motif de signal est déterminé sur la base de i données de sortie (i étant un nombre naturel supérieur ou égal à 2) des couches cachées.
